(19) Europäisches Patentamt
European Patent Office
Office européen des brevets

(11) Publication number: **0 050 492**
**B1**

(12) **EUROPEAN PATENT SPECIFICATION**

(45) Date of publication of patent specification: **15.08.84**

(21) Application number: **81304833.7**

(22) Date of filing: **16.10.81**

(51) Int. Cl.³: **C 07 D 471/14,**
C 07 D 471/22
//(C07D471/14, 221/00,
221/00, 209/00),
(C07D471/22, 221/00,
221/00, 221/00, 209/00)

# (54) 1,1-Disubstituted-hexahydro-indolo(2,3-a)quinolizine derivatives and their use in the enantioselective synthesis of optically active 14-oxo-E-homo-eburnane derivatives.

(30) Priority: **17.10.80 HU 252580**

(43) Date of publication of application:
**28.04.82 Bulletin 82/17**

(45) Publication of the grant of the patent:
**15.08.84 Bulletin 84/33**

(84) Designated Contracting States:
**BE CH DE FR GB LI NL**

(56) References cited:
DE - A - 2 167 043
DE - A - 2 625 672
GB - A - 2 010 833
GB - A - 2 028 809
GB - A - 2 051 794

(73) Proprietor: **RICHTER GEDEON VEGYESZETI GYAR R.T.**
**Gyömröi ut 19-21**
**H-1103 Budapest X (HU)**

(72) Inventor: **Szantay, Csaba**
**8, Zsombolyai u.**
**H-1113 Budapest (HU)**
Inventor: **Szabo, Lajos**
**38/b, Visegradi u.**
**H-1132 Budapest (HU)**
Inventor: **Kalaus, Gyorgy**
**3/E Bartok B. u.**
**H-1225 Budapest (HU)**
Inventor: **Sapi, Janos**
**2 Fadrusz u.**
**H-1114 Budapest (HU)**
Inventor: **Kreidl, Janos**
**51, Pusztaszeri u.**
**H-1025 Budapest (HU)**
Inventor: **Farkas, Maria**
**11, Magyar u.**
**H-1212 Budapest (HU)**
Inventor: **Nemes, Andras**
**11, Hankoczi u.**
**H-1022 Budapest (HU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 050 492 B1

(72) Inventor: Czibula, Laszlo
35, Kertesz u.
H-1073 Budapest (HU)

(74) Representative: Woodman, Derek et al,
FRANK B. DEHN & CO. Imperial House 15-19
Kingsway
London WC2B 6UZ (GB)

**Description**

This invention relates to new 1,1-disubstituted-1,2,3,4,6,7-hexahydro-12H-indolo[2,3-a]-quinolizinium salts, to processes for their preparation and to their use in the enantioselective synthesis of optically active 14-oxo-E-homo-eburnane derivatives of formula (Ia)

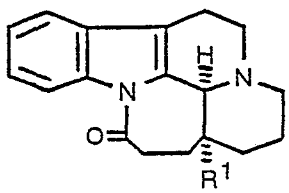

(Ia)

wherein $R^1$ represents a $C_{1-4}$ alkyl group.

The optically active compounds of general formula (Ia) are known in the art and may be used in the synthesis of (+)-vincamine and (+)-apovincaminic acid ethyl ester (Cavinton[R]), which have celebral vasodilating activity.

There are numerous processes known in the art for the preparation of (+)-vincamine and (+)-apovincaminic acid ethyl ester [see e.g. JACS, 1540 (1979) and British Patent Specification No. 2,010,833]. In most of them one of the racemic intermediates is resolved and the desired antipode obtained is subjected to the subsequent synthesis steps [see e.g. British Patent Specification No. 2,028,809 and DE 2625672]. This resolution step, however, results in a loss of at least 50% in yield. It would therefore be desirable to provide an enantioselective synthesis for the production of (+)-vincamine and/or -apovincaminic acid ethyl ester which does not require such a resolution step.

Efforts have already been made to devise an asymmetric synthesis of (+)-vincamine [Acta Chim. Hung. *99,* 73 (1979)]. In the process reported in the cited article naturally occuring L-tryptophan was employed as a starting material. The starting compound was first converted into its isopropyl ester whereafter the obtained L-tryptophan isopropyl ester was reacted with an ethylvaleric acid chloride derivative. The resultant cholopentanoyltryptophan isopropyl ester was subjected to ring closure by means of phosphorus oxychloride in benzene and was then treated with a mild base in methylene chloride to yield the perchlorate of optically active ethyl 1-ethyl-hexahydroindoloquinolizine-6-carboxylate of the general formula (II). Before the subsequent step of electrophilic alkylation with an acrylic acid ester derivative, however, the corresponding free base had to be liberated from the perchlorate. This treatment was carried out in methylene chloride, with a 2% aqueous sodium hydroxide solution. The product obtained after this treatment proved to be racemic. Accordingly, the conclusion can be drawn that in a higher pH range the optical activity of the carbon atom next to the ester group is lost, while at a lower pH-value the base cannot be set free from its perchlorate. While the electrophilic alkylation on the 1-carbon atom of the indolo[2,3-a]quinolizine skeleton can be performed only if the free base is used, the liberation of the free base from the corresponding indolo-quinolizinium salt is unavoidable.

We have surprisingly found that a chiral indolo[2,3-a]quinolizinium salt may be alkylated in the presence of a catalytic amount of a strong base, preferably an alkali metal alcoholate, such as potassium tert.butylate without attacking the chiral centre present. Hitherto, the use of a catalytic amount of strong bases during alkylation has been reported only in connection with non-chiral compounds [Tetrahedron 34, 3001—3004 (1978) and Hungarian Patent Application RI—713]. Using this alkylation method the addition reaction may be accomplished by reacting a hexahydroindoloquinolizinium salt with a corresponding, reactive olephin, such as acrolein or methylenemalonic acid ester.

According to the present invention the compounds of general formula (Ia) may be prepared by reacting an optically active 6-alkoxy-carbonyl-hexahydroindoloquinolizinium salt of formula (II),

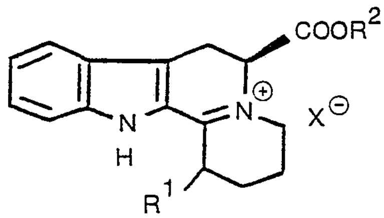

(II)

wherein

$R^1$ is as defined above,

$R^2$ represents a $C_{1-4}$ alkyl group and

X represents a residue of an acid, with a methylenemalonic acid diester derivative of formula (III)

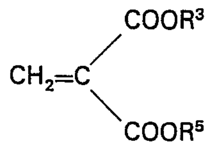

(III)

(wherein $R^3$ and $R^5$, which may be the same or different, each represents a $C_{1-4}$ alkyl group), in the presence of a strong basic catalyst. The optically active 6-alkoxy-carbonyl-hexahydroindoloquinolizinium-1-yl-methylene-malonate salt of formula (IV),

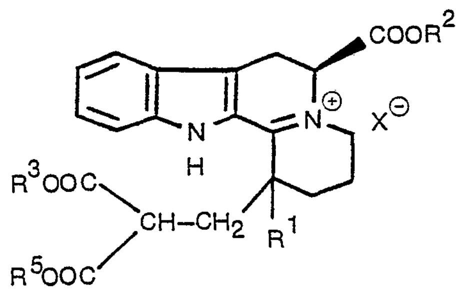

(IV)

(wherein $R^1$, $R^2$, $R^3$, $R^5$ and X are as defined above) thus obtained may then be subjected to acid hydrolysis followed by an alkaline hydrolysis to give an optically active hexahydroindoloquinolizinium-carboxylic acid derivative of formula (V),

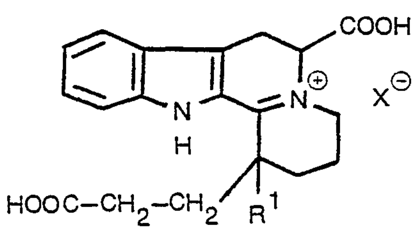

(V)

(wherein $R^1$ and X are as defined above) which is subsequently subjected to selective decarboxylation to give an optically active hexahydroindoloquinoliziummonocarboxylic acid of formula (VI)

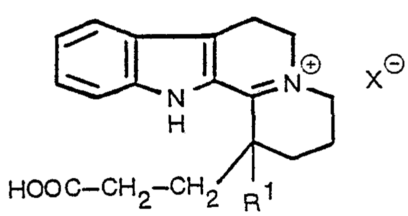

(VI)

(wherein $R^1$ and X are as defined above). The said compound of formula (VI) may then *either* be saturated to give an optically active octahydroindoloquinolizinemonocarboxylic acid derivative of formula (VII)

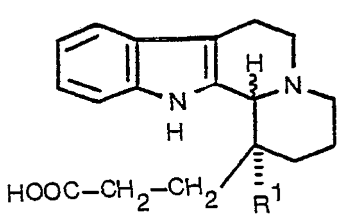

(VII)

(wherein $R^1$ is as defined above) which may then, optionally without isolation, be cyclised to give the

desired compound of formula (Ia) *or* the compound of formula (VI) may be esterified to give a compound of formula (VIa)

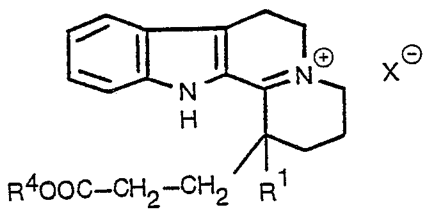

(VIa)

(wherein X, $R^1$ and $R^4$ are as defined above) which is subsequently saturated to give a compound of formula (VIII),

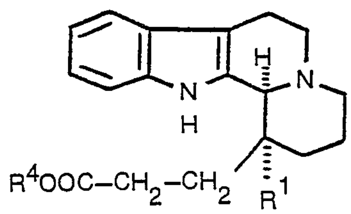

(VIII)

(wherein $R^1$ and $R^4$ are as defined above) which may then be cyclised, in a manner known *per se,* to the desired compound of formula (Ia). According to an alternative embodiment the compound of formula VIII may be obtained by esterification of a compound of formula (VII).

The intermediates of general formula (IV), (V) and (VI) are all new compounds which constitute, together with the individual steps of their synthesis, features of the present invention. These novel intermediates may be described by the general formula (A)

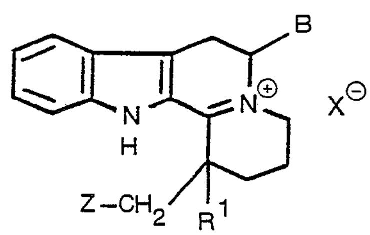

(A)

wherein

$R^1$ represents a $C_{1-4}$ alkyl group;

X represents a residue of an acid; and

*either* B represents a group ◄$COOR^2$ (in which $R^2$ represents a $C_{1-4}$ alkyl group) and Z represents a group

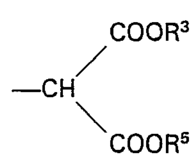

(in which $R^3$ and $R^5$, which may be the same or different, each represents a $C_{1-4}$ alkyl radical) *or* B represents a group —COOH or a hydrogen atom and Z represents a group —$CH_2COOH$.

In particular there may be mentioned the following:

(—)-1$\alpha$-ethyl-1$\beta$-(2′,2′-diethoxycarbonylethyl)-6$\beta$-methoxycarbonyl-1,2,3,4,6,7-hexahydro-12H-indolo[2,3-a]quinolizin-5-ium perchlorate,

(+)-1$\alpha$-ethyl-1$\beta$-carboxyethyl-6-carboxy-1,2,3,4,6,7-hexahydro-12H-indolo[2,3-a]quinolizin-5-ium perchlorate and

(—)-1$\alpha$-ethyl-1$\beta$-carboxyethyl-1,2,3,4,6,7-hexahydro-12H-indolo[2,3-a]quinolizin-5-ium perchlorate.

As starting materials of the process according to the invention the intermediates described in Hungarian Patent Application 781/80 are used. These compounds are characterized by the general

formula (II) and have a considerable optical rotatory power.

In a process disclosed in Hungarian Patent Application No. 781/80 it was attempted to produce the E ring of the eburnane skeleton from a chiral starting material, using the highly reactive acrolein as an electrophilic alkylating agent. By this method, however, the product could be obtained in an optical purity of about 62% only.

The optically active starting compounds of general formula (II) can be prepared by reacting an optically active tryptophan ester derivative with an alkylvaleric acid halide in the presence of an acid binding agent and subjecting the optically active halopentanoyltryptophan ester derivative obtained to simultaneous cyclisation and dehydration followed by treatment with a base.

The optically active starting compounds of general formula (II) contain a centre of chirality at the site of attachment of the carboxyl group. This centre of chirality preserves the optical activity of the the optically active tryptophan ester from which compound has been prepared until a new centre of chirality is formed in the molecule in a configuration corresponding to the desired end product. After this the carboxyl group which is not needed in the end product and only served to preserve the optical activity can be eliminated at any appropriate time. Although when the carboxyl group is eliminated, the centre of chirality at the carbon atom to which it has been attached ceases to exist, the molecule already has the desired optical activity.

In the process according to the invention the reaction of the starting compound of the general formula (II) with methylenemalonic acid diester derivatives of the general formula (III) (which are even more reactive than acrolein) can be completed in an unusually short time, i.e. in about half to one hour and consequently, the end product may be obtained in an optical purity exceeding 90%. This means that during the addition practically no racemisation takes place or, in other words, the reaction velocity of the addition of methylenemalonic acid ester is much higher than the velocity of racemisation. Optical purity was determined in comparison with compounds of general formulae (Ia) and (VIII), which have a known absolute optical rotation.

The preparation of the methylenemalonic acid esters of the general formula (III) is described in J. Org. Chem. *4*, 493 (1939).

The compounds of general formula (II) are conveniently reacted with the compounds of general formula (III) in an inert organic solvent such as, for example an aliphatic or aromatic hydrocarbon optionally substituted by one or more halogens, e.g. dichloromethane, dichloroethane, chloroform or chlorobenzene and preferably under anhydrous conditions. The reaction is performed in the presence of a strong basic catalyst preferably an alkali metal tertiary alcoholate, most preferably potassium tert.-butylate. Convenient temperatures for the reaction are from 0 to 35°C, preferably 10 to 25°C. It is preferred to use freshly prepared compounds of general formula (III) in a molar excess of about 1.1 to 2, preferably 1.1 to 1.4 relative to the compounds of general formula (II). Alkali metal tertiary alcoholates are generally used in an amount of 3 to 7 molar %, preferably 4 to 6 molar % relative to the compounds of general formula (II). The reaction is generally completed in about half to one hour, with a nearly quantitative yield. The new compounds of general formula (IV) are obtained as an oil and may be characterized by their optical rotation.

The acid hydrolysis of the compounds of general formula (IV) is generally accomplished with a dilute aqueous mineral acid, preferably with a mineral acid easy to eliminate when evaporating the reaction mixture, e.g. a dilute aqueous hydrochloric acid solution. The acid hydrolysis is conveniently performed at an elevated temperature, preferably about the boiling temperature of the reaction mixture (80 to 120°C). The alkaline hydrolysis is preferably carried out with an aqueous solution of an alkali metal hydroxide, most preferably sodium hydroxide. As a co-solvent alkanols having 1 to 4 carbon atoms, preferably ethanol can be employed. After eliminating the alkanols, the alkali metal salts obtained during the neutralisation following the hydrolysis are readily dissolved in the aqueous solution and accordingly do not contaminate the new, optically active compounds of general formula (V) obtained. Alkaline hydrolysis is preferably performed at room temperature (10 to 35°C).

The selective decarboxylation of the compounds of general formula (V) is generally carried out by heating, at a temperature of from 150°C to 200°C, preferably 165°C to 175°C, preferably in an inert organic solvent having a high boiling point. As a solvent any inert organic solvents having a boiling point of from 150°C to 200°C may be employed, e.g. decalin, tetralin, quinoline or isoquinoline. Decalin is particularly preferred.

The compounds of general formula (VI) may be saturated with catalytically activated hydrogen or a chemical reducing agent. When the saturation is performed with catalytically activated hydrogen, metals, e.g. palladium, platinum, nickel, iron, copper, cobalt, chromium, zinc, molybdenum, wolfram and their oxides may be employed as a hydrogenating catalyst. Catalytic hydrogenation may be carried out also in the presence of catalysts which have previously been precipitated on the surface of a carrier. Such carriers include carbon, preferably charcoal, silica, alumina and sulfates and carbonates of alkali earth metals. Catalytic hydrogenation is preferably accomplished in an inert solvent, such as water, aqueous alkaline solutions, alcohols, ethyl acetate, dioxan, glacial acetic acid, dimethylacetamide, dimethylformamide or mixtures thereof. As a chemical reducing agent for example complex metal hydrides, e.g. borohydrides, such as alkali metal borohydrides, preferably sodium borohydride or aluminium hydrides such as lithium aluminium hydride can be employed. Chemical reduction is

preferably performed in an inert solvent such as water, aqueous alcohol or acetonitrile. Preferred solvents or suspending agents include aliphatic alcohols, e.g. methanol.

Following the process according to the invention compounds of general formula (VI) are most preferably saturated by catalytic hydrogenation in the presence of a palladium-on-charcoal catalyst, in an inert organic solvent, such as dimethylformamide. As a result of catalytic hydrogenation an epimeric mixture of compounds of general formula (VII) is obtained, containing the cis isomer in an overwhelming majority. When hydrogenation is accomplished in the presence of a palladium-on-charcoal catalyst, in dimethylformamide, C/D cis annelated 1α-alkyl-12αH-octahydroindoloquinolizines are obtained stereoselectively and the corresponding trans 12bβH-epimers are obtained only as by-products. If desired, the two epimers of general formula (VII) can be separated, but their separation is not required for the subsequent step of synthesis.

Cyclisation of the compounds of general formula (VII) may be carried out, for example, with a phosphorus halide, phosphorus oxyhalide or sulfur oxyhalide, such as e.g. phosphorus pentachloride, phosphorus pentabromide or thionyl chloride. Phosphorus oxychloride is particularly preferred. The reaction temperature is generally from 10°C to 100°C, preferably 15°C to 30°C. The cyclisation may also be performed at a temperature of 60 to 80°C for one to 3 hours. By this method compounds of general formula (Ia) may be obtained with a good yield, in an optical purity of about 98%.

The process according to the invention is preferably carried out by subjecting the cis-trans mixture of the compounds of general formula (VII), obtained by saturating compounds of general formula (VI) to cyclisation without isolation from the reaction mixture, thereby reducing the reaction steps of the overall synthesis. When using this preferred embodiment, the total yield calculated for the starting compounds of general formula (II) amounts to about 35%. The optical purity of the compounds of general formula (Ia) obtained is about 96%. The major product of the cyclisation is the compound of formula (Ia). However a few percent of a cis-trans isomeric mixture of general formula (Ib)

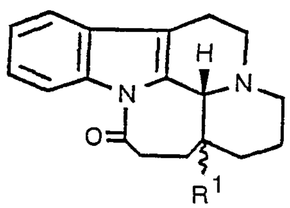

(Ib)

may also be isolated as a by-product from the mother liquor.

According to another embodiment of the process according to the invention the acids of general formula (VI) may first be converted into their corresponding esters of formula (VIa) which are then saturated, or alternatively, they are saturated and then the acids of general formula (VII) obtained are converted into the corresponding esters. By both routes octahydroindoloquinolizine ester derivatives of general formula (VIII) are obtained, which may then be cyclized in a known manner, for example by means of sodium ter.-butylate (Hungarian Patent Specification No. 163,769 and DE 2167043). The esterification of the compounds of general formula (VI) and (VII), respectively may be carried out as described in British Patent Specification No. 1,548,671 for the esterification of certain compounds of general formula (VII). When the methyl esters are to be prepared, the corresponding acids are preferably reacted with diazomethane in an inert organic solvent, such as halogenated hydrocarbons, preferably dichloromethane.

Compounds of the general formula (Ia) prepared according to the invention, if desired, may be subjected to further purification, e.g. recrystallization.

The new enantioselective synthesis for the preparation of the compounds of general formula (Ia), which are useful intermediates in the preparation of pharmaceutically active alkaloids having an eburnane skeleton, provides the desired products with a very good yield, in a high optical purity. The reactions involved are very easy to carry out. A further advantage of the process according to the invention lies in the fact that, by using the extremely reactive compounds of general formula (III), racemisation is practically impossible. In the second step of the synthesis by acid and a subsequent alkaline hydrolysis of the compounds of the general formula (IV) dicarboxylic acids of the general formula (V) can be prepared, from which the functional carboxyl group preserving the original chirality can be eliminated by selective decarboxylation, without any difficulty. After stereoselective saturation of the C=N bond of the new carboxylic acids of the general formula (VI) the compounds obtained can directly be transformed into the desired compounds of the general formula (Ia), and the conversion can be performed with an excellent yield, giving the compounds of the general formula (Ia) in a high optical purity.

Further details of the invention are illustrated by the following Examples, which are not intended to limit the scope of invention.

Example 1

(—)-1α-Ethyl-1β-(2',2'-diethoxycarbonylethyl)-6β-methoxycarbonyl-1,2,3,4,6,7-hexahydro-12H-indolo[2,3-a]quinolizin-5-ium perchlorate

200 g (4.87 mmoles) of (+)-1-ethyl-6β-methoxycarbonyl-1,2,3,4,6,7-hexahydro-12H-indolo-[2,3-a]quinolizin-5-ium perchlorate ($[\alpha]_{546}^{22} = +135°$, c=1, dichloromethane) are dissolved in 15 ml of absolute dischloromethane whereafter 1.10 ml (1.16 g., 6.75 mmoles) of freshly prepared methylenemalonic acid diethyl ester [J. Org. Chem. *4*, 493 (1939)] are added to the solution, with stirring. To the reaction mixture cooled to 0°C a suspension of 30 mg (0.268 mmoles) of potassium tert.-butylate in 5 ml of dichloromethane is added under stirring. At room temperature the reaction is completed within one hour. After neutralisation with one drop of hydrochloric acid in ethanol, the solvent is eliminated from the reaction mixture *in vacuo*. The residue is then triturated with two 8 ml portions of ether, the ether is decanted and the oily product is released from ether *in vacuo*. 2.75 g of the title compound are obtained as an oily product.

Yield 96.5%
$[\alpha]_D^{23} = -10°$; $[\alpha]_{546}^{23} = -17°$ (c = 1, dichloromethane)
IR spectrum (KBr): 3400 (indole NH), 1745, 1730 (ester CO groups) $cm^{-1}$

Example 2

(+)-1α-Ethyl-1β-carboxyethyl-6-carboxy-1,2,3,4,6,7-hexahydro-12H-indolo[2,3-a]quinolizin-5-ium perchlorate

1.50 g (2.57 mmoles) of (—)-1α-ethyl-1β-(2'-diethoxycarbonylethyl)-6β-methoxycarbonyl-1,2,3,4,6,7-hexahydro-12H-indolo[2,3-a]quinolizin-5-ium perchlorate prepared in Example 1 are dissolved in 10 ml of ethanol. To the solution 15 ml of a 10% aqueous hydrochloric acid solution are added and the mixture obtained is refluxed for 24 hours.

The solvent is eliminated from the reaction mixture *in vacuo* and the residue is triturated with 5 ml of ether whereafter ether is eliminated *in vacuo.*

The oily residue is dissolved in 30 ml of ethanol and to the solution a solution of 0.62 g (15.45 mmoles) of sodium hydroxide in 3 ml of water is added. At room temperature the reaction terminates in 6 hours.

The solvent is eliminated from the reaction mixture *in vacuo.* The oily residue is dissolved in 7 ml of water and the pH of the solution is adjusted to 3 with a 70% perchloric acid solution, under cooling with ice water. The yellow precipitate is filtered off, washed with 2 ml of water and dried. 1.10 g of the title compound are obtained, melting at 183°C (decomp.).

Yield: 92.0%
$[\alpha]_D^{23} = +36°$; $[\alpha]_{546}^{23} = +47.5°$ (c = 0.8, methanol)
IR spectrum (KBr): 3450—3300 (OH, indole-NH), 1715, 1620 (carboxylate), 1610 (C=N) $cm^{-1}$

Example 3

(—)-1α-Ethyl-1β-carboxyethyl-1,2,3,4,6,7-hexahydro-12H-indolo[3,2-a]quinolizin-5-ium perchloride

1000 mg. (2.13 mmoles) of (+)-1α-ethyl-1β-carboxyethyl-6-carboxy-1,2,3,4,6,7-hexahydro-12H-indolo[2,3-1]quinolizin-5-ium perchloride in 15 ml. of decalin are heated at 160 to 170°C for 25 minutes, with stirring. The substance melts accompanied by gas evolution. After cooling the reaction mixture is filtered, the solid obtained is washed decalin-free with three 5-ml. portions of ether and is dried. 880 mg. of the title compound are obtained.

Yield 97.2%
$[\alpha]_D^{25} = -19°$
IR spectrum (KBr): 3350 (indole NH), 1710, 1620 (carboxylate), 1580, 1520 (C=N) $cm^{-1}$

Example 4

(—)-1α-Ethyl-1β-methoxycarbonylethyl -1,2,3,4,6,7,12,12b-octahydro-indolo[2,3-a]quinolizine

600 mg. (1.55 mmoles) of (—)-1α-ethyl-1β-carboxyethyl-1,2,3,4,6,7-hexahydro-12H-indolo-[2,3-a]quinolizin-5-ium perchlorate prepared in Example 3 are dissolved in 10 ml. of absolute dimethylformamide and subsequently hydrogenated in the presence of 800 mg. of a prehydrogenated 10% palladium-on-charcoal catalyst.

When the calculated amount of hydrogen has been taken up, the catalyst is filtered off, washed with two 5-ml. portions of dimethylformamide and the combined organic phases are evaporated to dryness under a pressure of 1 to 2 mmHg.

The oily residue is dissolved in a mixture of 15 ml. of dichloromethane and 5 ml. of methanol and a solution of an excess amount of diazomethane in dichloromethane is added with external ice cooling and stirring [A. Vogel: Practical Organic Chemistry 3rd Edition, p. 971]. The progress of the reaction is monitored by thin layer chromatography (KG—G, a 14:3 mixture of benzene and methanol).

The solvent is eliminated from the reaction mixture *in vacuo.* The residue is dissolved in 20 ml. of

dichloromethane and the base is liberated by shaking the solution with 5 ml. of a 10% aqueous sodium carbonate solution. After a further extraction with 5 ml. of dichloromethane the organic phases are separated, combined, dried over anhydrous magnesium sulfate, filtered and the solvent is eliminated *in vacuo.*

610 mg. of an oily product are obtained containing the title compound at a main component.

The mixture is separated into three components by preparative thin layer chromatography (KG—$PF_{254+366}$, a 140:30 mixture of benzene and methanol, elution:acetone). The substance having the lowest $R_f$-value weighs 240 mg.

Yield: 45.5% of the title compound
For the crude product $[\alpha]_D^{21} = -92°$, $[\alpha]_{546}^{21} = -110°$ (c = 2.14, dichloromethane)

240 mg. of the oily product are dissolved in 0.5 ml. of methanol and the pH of the solution is adjusted to 6 with hydrochloric acid in methanol. The precipitated crystals are filtered off and dried. 180 mg. of the title compound are obtained as the hydrochloride salt.

Yield: 40.0%
$[\alpha]_D^{22} = -118°$; $[\alpha]_{546}^{22} = -143°$ (c = 1.38, dichloromethane)

The optical rotatory powers correspond to the free base.

The maximum value measured up to now is $[\alpha]_D^{25} = -121°$ (c = 2.02, dichloromethane, Hungarian Patent Application No. RI—633).

IR spectrum (KBr): 3400 (indole NH), 1735 (ester CO) $cm^{-1}$.

The $^1H$—NMR and MS spectra of the compound are identical with the corresponding spectra of the compound prepared according to Hungarian Patent Application RI—633.

As a by-product 50 mg. (10.5%) of (+)-14-oxo-E-homoeburnane are obtained as an oily product which can be easily isolated.

Of the component having the highest $R_f$-value 35 mg. 7.3% could be isolated as an oily product which was identified as (+)-14-oxo-3-epi-E-homoeburnane.

$[\alpha]_D^{24} = +61°$ (c= 1, dichloromethane).

The $R_f$-values decrease in the following order: (+)-14-oxo-3-epi-E-homo-eburane >(+)-14-oxo-E-homo-eburnane >(—)-1$\alpha$-ethyl-1$\beta$-methoxycarbonylethyl-1,2,3,4,6,7,12,12b-octahydro-indolo[2,3-a]-quinolizine (KG—G, a 14:3 mixture of benzene and methanol).

## Example 5

(+)-14-Oxo-E-homo-eburnane(3$\alpha$,17$\alpha$)

400 mg. (0.943 mmoles) of (—)-1$\alpha$ethyl-1$\beta$-carboxyethyl-1,2,3,4,6,7-hexahydro-12H-indolo-[2,3-a]quinolizin-5-ium perchlorate prepared in Example 3 are dissolved in 9 ml. of absolute dimethylformamide and hydrogenated in the presence of 800 mg of a prehydrogenated 10% palladium-on-charcoal catalyst.

When the calculated amount of hydrogen is taken up, the catalyst is filtered off from the reaction mixture. The catalyst is then washed with two 5-ml. portions of dimethylformamide and the combined organic phases are evaporated to dryness on a bath of 50 to 60°C, under a pressure of 1 to 2 mmHg.

The oily residue is dissolved in 3 ml. of phosphorus oxychloride under cooling with ice and the solution is kept at room temperature for two or three days.

5 ml. of benzene are added to the reaction mixture and the unreacted phosphorus oxychloride is eliminated by distillation *in vacuo.* 5 ml. of water are added to the residue under cooling with ice, and then the pH is adjusted to 9 with a concentrated aqueous ammonium hydroxide solution. The organic precipitate is extracted with three 8 ml. portions of dichloromethane. The combined organic phases are dried over anhydrous magnesium sulfate, filtered and the solvent is distilled off *in vacuo.*

As a distillation residue 420 mg. of an oily product are obtained, which are then subjected to preparative thin layer chromatography (KG—$PF_{254+366}$, a 140:30 mixture of benzene and methanol, elution:acetone).

The substance having the lower $R_f$-value is the title compound, weighing 129 mg. (oily).

Yield: 44.4%
$[\alpha]_D^{24} = +13°$, $[\alpha]_{546}^{24} = +19°$ (c = 2, dichloromethane).

100 mg. of the oily product are crystallized from 0.5 ml. of methanol to yield 117 mg. (40%) of the title compound in a crystalline form.

$[\alpha]_D^{24} = +12°$, $[\alpha]_{546}^{24} = +18°$ (c = 1, dichloromethane)

Melting point: 150°C (methanol) (literature: 151°C, see Hungarian Patent Specification No. 163,769)
IR spectrum (KBr): 1700 $cm^{-1}$ (lactam)

The optical purity was determined on (—)-1$\alpha$-ethyl-1$\beta$-methoxycarbonylethyl-1,2,3,4,6,7,12,12b$\alpha$-octahydro-indolo[2,3-a]quinolizine prepared by opening the lactam ring of (+)-14-oxo-E-homo-eburnane (3$\alpha$,17$\alpha$) as described below:

50 mg. of (+)-14-oxo-E-homo-eburnane (3$\alpha$,17$\alpha$) in a solution of 25 mg. of potassium tert.-butylate in 1 ml. of methanol are allowed to stand at room temperature for 2 to 3 hours. The progress of the reaction is monitored by thin layer of chromatography. (The $R_f$-value of the starting compound is higher than that of the product obtained by opening the ring: KG—G, a 14:3 mixture of benzene and methanol.)

The reaction mixture is treated with 1 to 2 drops of acetic acid under cooling with ice and the solvent is eliminated *in vacuo.* The residue is dissolved in 2 ml. of dichloromethane, the pH of the solution is adjusted to 9 with 1 ml. of a 5% aqueous sodium carbonate solution, the organic phase is separated and the extraction is repeated with a further 2-ml. portion of dichloromethane. The organic phases are combined, dried over anhydrous magnesium sulfate, filtered and the solvent is distilled off from the filtrated *in vacuo.* As a distillation residue 58 mg. of (—)1$\alpha$-ethyl-1$\beta$-methoxycarbonylethyl-1,2,3,4,6,7,12,12b$\alpha$-octahydro-indolo[2,3-a]quinolizine are obtained as an oily substance.

$[\alpha]_D^{22} = -116°$, $[\alpha]_{546}^{22} = -40°$ (c = 1.16, dichloromethane)

Optical purity:

$$\frac{-116°}{-121°} = 0.96 = 96\%$$

The chromatographically isolated product having a higher $R_f$-value weighs 41 mg. (14.1%) and is identified as (+)-1$\alpha$-14-oxo-3-epi-E-homo-eburnane.

The optical rotatory power of the crude oil is as follows: $[\alpha]_D^{22} = +107°$; $[\alpha]_D^{22} = +128°$ (c = 1.0, dichloromethane).

The oil product, weighing 41 mg. is crystallized from 0.3 ml. of methanol to yield 31 mg. of crystalline (+)-14-oxo-3-epi-E-homo-eburnane, melting at 110 to 112°C (ethanol). [The melting point of this product according to Hungarian Patent Application No. 781/80 is 121 to 122°C (isopropanol)].

The IR, MS and NMR data of the compound are identical with the characteristics of the corresponding racemic compound (see Hungarian Patent Application RI—634).

Example 6

(+)-14-Oxo-E-homo-eburnane(3$\alpha$,17$\alpha$)

100 mg. (0.307 mmoles) of (—)-1$\alpha$-ethyl-1$\beta$-(hydroxycarbonyl-ethyl)-1,2,3,4,6,7,12,12b$\alpha$-octahydro-indole[2,3-a]-quinolizine, prepared according to the British Patent Specification No. 1,548,671, in 1 ml. of phosphorus oxychloride are allowed to stand at room temperature for 2 days. 3 ml. of benzene are added to the reaction mixture, whereafter the unreacted phosphorus oxychloride and benzene are distilled off *in vacuo.*

To the distillation residue 1 ml. of water is added and the pH is adjusted to 9 with a concentrated aqueous ammonium hydroxide solution, under cooling with ice. The precipitated organic substance is extracted with three 2 ml. portions of dichloromethane and the organic phases are combined, dried over anhydrous magnesium sulfate, filtered and the solvent is distilled off *in vacuo.*

92 mg. of an oily distillation residue are obtained, which are crystallized from 1 ml. of ether to yield 76 mg. of the title compound.

Yield: 80.0%
Melting point: 154 to 155°C (ether)
IR spectrum (KBr): 1700 $cm^{-1}$ (lactam)
$[\alpha]_D^{23} = +19°$; $[\alpha]_{546}^{23} = +22°$ (c = 1, dichloromethane)

The optical purity is determined on (—)1$\alpha$-ethyl-1$\beta$-methoxycarbonylethyl-1,2,3,4,6,7,12,12b$\alpha$-octahydro-indolo[2,3-a]quinolizine prepared by opening the lactam ring of (+)-14-oxo-E-homo-eburnane(3$\alpha$,17$\alpha$), as described in Example 5.

The optical rotatory power of (—)-1$\alpha$-ethyl-1$\beta$-methoxy-carbonylethyl-1,2,3,4,6,7,12,12b$\alpha$-octahydro-indolo[2,3-a]quinolizine is:

$[\alpha]_D^{23} = -118°$; $[\alpha]_D^{23} = -141°$ (c = 1.06, dichloromethane)

Optical purity:

$$\frac{-118°}{-121°} = 0.98 = 98\%$$

The MS and NMR spectra of the compound as well as its physical and chemical properties are identical with the corresponding properties of the substance disclosed in Hungarian Patent Specification 163 769.

## Claims

1. Compounds of general formula (A),

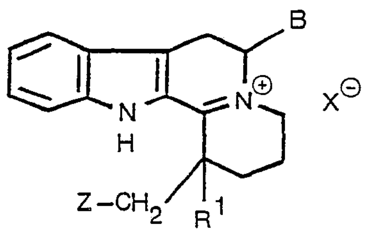

(A)

wherein

$R^1$ represents a $C_{1-4}$ alkyl group;

X represents a residue of an acid;

*either* B represents a group ◄$COOR^2$ (in which $R^2$ represents a $C_{1-4}$ alkyl group) and Z represents a group

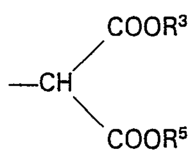

(in which $R^3$ and $R^5$, which may be the same or different, each represents a $C_{1-4}$ alkyl radical) *or* B represents a group —COOH or a hydrogen atom and Z represents a group $—CH_2COOH$.

2. Compounds as claimed in claim 1 having the general formula (IV),

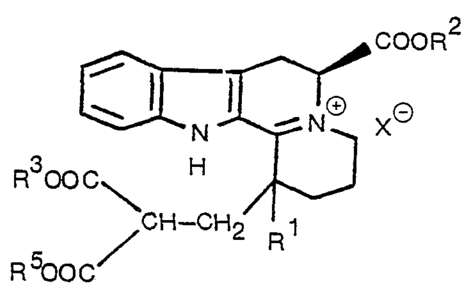

(IV)

wherein $R^1$, X, $R^2$, $R^3$ and $R^5$ are as defined in claim 1.

3. Compounds as claimed in claim 1 having the general formula (V),

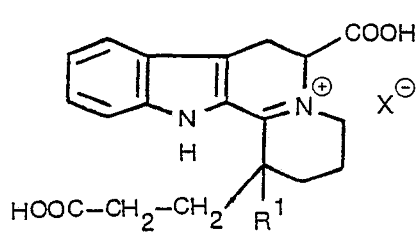

(V)

wherein $R^1$ and X are as defined in claim 1.

4. Compounds as claimed in claim 1 having the general formula (VI),

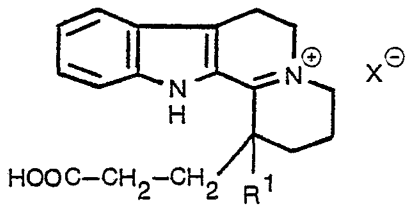

(VI)

wherein R′ and X are as defined in claim 1.

5. A compound as claimed in claim 1 selected from the following:

(—)-1α-ethyl-1β-(2′,2′-diethoxycarbonylethyl)-6β-methoxycarbonyl-1,2,3,4,6,7-hexahydro-12H-indolo[2,3-a]quinolizin-5-ium perchlorate,

(+)-1α-ethyl-1β-carboxyethyl-6-carboxy-1,2,3,4,6,7-hexahydro-12H-indolo[2,3-a]quinolizin-5-ium perchlorate and

(—)-1α-ethyl-1β-carboxyethyl-1,2,3,4,6,7-hexahydro-12H-indolo[2,3-a]quinolizin-5-ium perchlorate.

6. A process for the preparation of compounds of general formula (IV) as defined in claim 2 which comprises reacting an optically active 6-alkoxy-carbonyl-hexahydroindoloquinolizinium salt of formula (II),

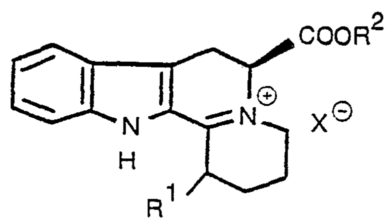

(II)

(wherein $R^1$, X and $R_2$ are defined as claimed 1) with a methylenemalonic acid diester of formula III,

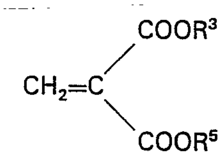

(III)

(wherein $R^3$ and $R^5$ are as defined in claim 1), in the presence of a strong basic catalyst.

7. A process for the preparation of compounds of general formula (V) as defined in claim 3 which comprises subjecting a compound of formula (IV) as defined in claim 2 to acid and then alkaline hydrolysis.

8. A process for the preparation of compounds of general formula (VI) as defined in claim 4 which comprises subjecting a compound of formula (V) as defined in claim 3 to selective decarboxylation whereby the desired compound of formula (VI) is obtained.

9. The use of a compound of formula (VI) as defined in claim 4 in a process for the preparation of compounds of general formula (VIII),

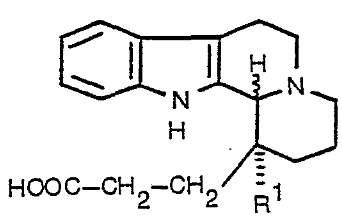

(VII)

(wherein $R^1$ is as defined in claim 1) which comprises saturating the compound of formula (VI) whereby the desired compound of formula (VII) is obtained.

10. A process as claimed in claim 9 wherein the compound of formula (VII) is subsequently cyclised to yield a compound of formula (Ia),

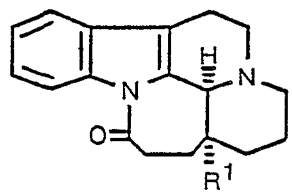

(Ia)

(wherein $R^1$ is as defined in claim 1).

11. The use of a compound of formula (VI) as defined in claim 4 in a process for the preparation of compounds of general formula (VIa),

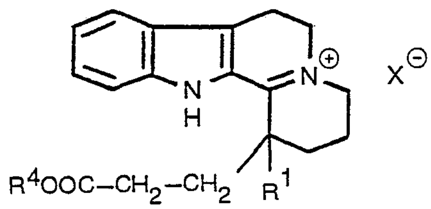

(VIa)

(wherein $R^1$ and X are as defined in claim 1 and $R^4$ represents a $C_{1-4}$ alkyl group) which comprises esterifying the compound of formula (VI).

12. A process as claimed in claim 11 wherein the compound of formula (VIa) is subsequently saturated to yield a compound of formula (VIII),

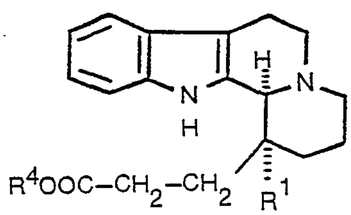

(VIII)

(wherein $R^1$ is as defined in claim 1 and $R^4$ is as defined in claim 11).

13. A process as claimed in claim 9 wherein the compound of formula (VII) is esterified to yield a compound of formula (VIII) as defined in claim 12.

14. A process as claimed in claim 12 or claim 13, wherein the compound of formula (VIII) is subsequently subjected to ring closure whereby a compound of formula (Ia) as defined in claim 10 is obtained.

**Patentansprüche**

1. Verbindungen der allgemeinen Formel (A),

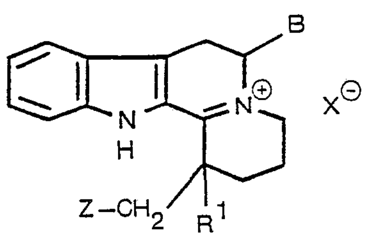

(A)

worin

$R^1$ eine $C_{1-4}$-Alkylgruppe,

X einen Säurerest und entweder B eine ◄COOR$^2$-Gruppe, worin $R^2$ einen $C_{1-4}$-Alkylrest bedeutet, und Z eine Gruppe der Formel

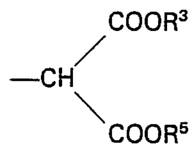

worin $R^3$ und $R^5$, die gleich oder verschieden sein können, jeweils für einen $C_{1-4}$-Alkylrest stehen, oder B eine —COOH-Gruppe oder ein Wasserstoffatom und Z eine —$CH_2COOH$-Gruppe bedeuten.

2. Verbindungen gemäß Anspruch 1 der allegemeinen Formel (IV),

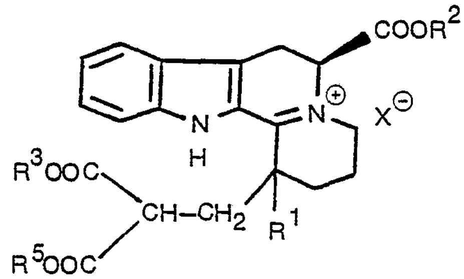

(IV)

worin $R^1$, X, $R^2$, $R^3$ und $R^5$ die in Anspruch 1 angegebene Bedeutung besitzen.

3. Verbindungen gemäß Anspruch 1 der allgemeinen Formel (V),

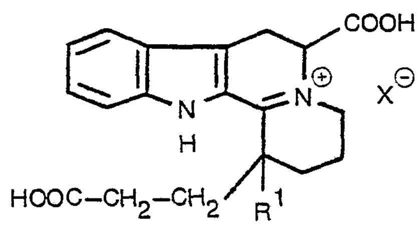

(V)

worin $R^1$ und X die in Anspruch 1 angegebene Bedeutung besitzen.

4. Verbindungen gemäß Anspruch 1 der allgemeinen Formel (VI),

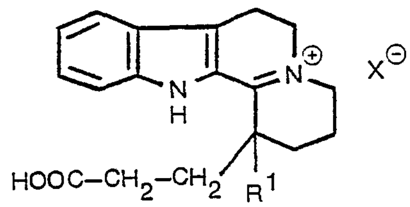

(VI)

worin $R^1$ und X die in Anspruch 1 angegebene Bedeutung besitzen.

5. Verbindung gemäß Anspruch 1 aus der Gruppe der folgenden Verbindungen:
(—)-1α-Ethyl-1β-(2',2'-diethoxycarbonylethyl)-6β-methoxycarbonyl-1,2,3,4,6,7-hexahydro-12H-indolo[2,3-a]chinolizin-5-ium-perchlorat,
(+)-1α-Ethyl-1β-carboxyethyl-6-carboxy-1,2,3,4,6,7 -hexyhydro-12H-indolo[2,3-a]chinolizin-5-ium-perchlorat und
(—)-1α-Ethyl-1β-carboxyethyl-1,2,3,4,6,7-hexahydro-12H-indolo[2,3-a]chinolizin-5-ium-perchlorat.

6. Verfahren zur Herstellung von Verbindungen der allgemeinen Formel (IV) gemäß Anspruch 2, dadurch gekennzeichnet, daß man ein optisch aktives 6-Alkoxy-carbonyl-hexahydroindolochinoliziniumsalz der Formel (II),

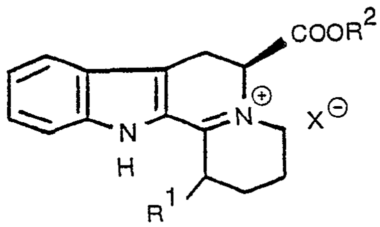

(II)

worin $R^1$, X und $R^2$ die in Anspruch 1 angegebene Bedeutung besitzen, mit einem Methylenmalonsäurediester der Formel (III),

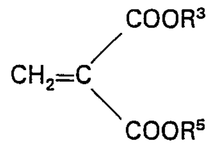

(III)

worin $R^3$ und $R^5$ die in Anspruch 1 angegebene Bedeutung besitzen, in Gegenwart eines starken basischen Katalysators umsetzt.

7. Verfahren zur Herstellung von Verbindungen der allgemeinen Formel (V) gemäß Anspruch 3, dadurch gekennzeichnet, daß man eine Verbindung der Formel (IV) gemäß Anspruch 2 der sauren und anschließend der alkalischen Hydrolyse unterwirft.

8. Verfahren zur Herstellung von Verbindungen der allgemeinen Formel (VI) gemäß Anspruch 4, dadurch gekennzeichnet, daß man eine Verbindung der Formel (V) gemäß Anspruch 3 der selektiven Decarboxylierung unterwirft, wodurch man die gewünschte Verbindung der Formel (VI) erhält.

9. Verwendung einer Verbindung der Formel (VI) gemäß Anspruch 4 in einem Verfahren zur Herstellung von Verbindungen der allgemeinen Formel (VII),

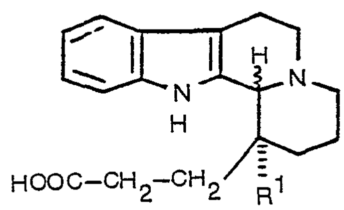

(VII)

worin $R^1$ die in Anspruch 1 angegebene Bedeutung besitzt, dadurch gekennzeichnet, daß man die Verbindung der Formel (VI) sättigt, wodurch man die gewünschte Verbindung der Formel (VII) erhält.

10. Verfahren gemäß Anspruch 9, dadurch gekennzeichnet, daß man die Verbindung der Formel (VII) anschließend zu einer Verbindung der Formel (Ia),

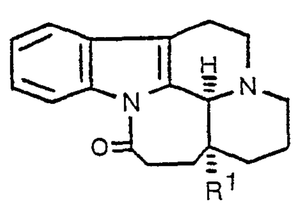

(Ia)

worin $R^1$ die in Anspruch 1 angegebene Bedeutung besitzt, cyclisiert.

11. Verwendung einer Verbindung der Formel (VI) gemäß Anspruch 4 in einem Verfahren zur Herstellung von Verbindungen der allgemeinen Formel (VIa),

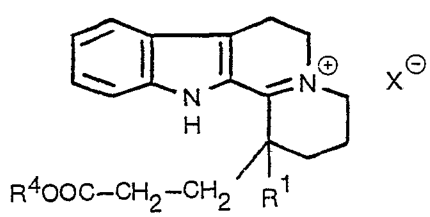

(VIa)

worin $R^1$ und X die in Anspruch 1 angegebene Bedeutung besitzt und $R^4$ eine $C_{1-4}$-Alkylgruppe bedeutet, dadurch gekennzeichnet, daß man die Verbindung der Formel (VI) verestert.

12. Verfahren gemäß Anspruch 11, dadurch gekennzeichnet, daß man die Verbindung gemäß Formel (VIa) anschließend zu einer Verbindung der Formel (VIII),

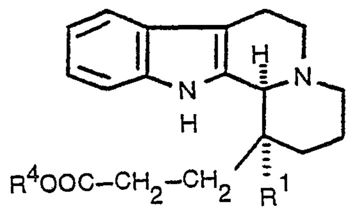

(VIII)

worin $R^1$ die in Anspruch 1 angegebene und $R^4$ die in Anspruch 11 angegebene Bedeutung besitzen, sättigt.

13. Verfahren gemäß Anspruch 9, dadurch gekennzeichnet, daß man die Verbindung der Formel (VII) zu einer Verbindung der Formel (VIII) gemäß Anspruch 12 verestert.

14. Verfahren gemäß Anspruch 12 oder 13, dadurch gekennzeichnet, daß man die Verbindung der Formel (VIII) anschließend einer Umsetzung unter Ringschluß zu einer Verbindung der Formel (Ia) gemäß Anspruch 10 unterwirft.

**Revendications**

1. Composés, caractérisés en ce qu'ils sont représentés par la formule générale (A) ci-après:

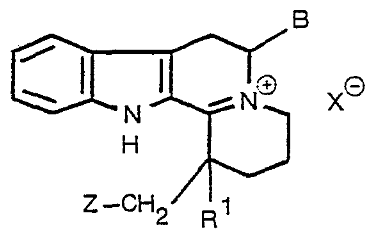

(A)

dans laquelle

$R^1$ est un groupe alcoyle en $C_{1-4}$;

X représente un résidu d'un acide;

soit B représente un groupe ◀ $COOR^2$ (dans lequel $R^2$ est un groupe alcoyle en $C_{1-4}$) et Z représente un groupe

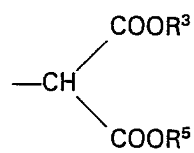

(dans lequel $R^3$ et $R^5$, qui peuvent être identiques ou différents, représentent chacun un radical alcoyle en $C_{1-4}$), soit B représente un groupe —COOH ou un atome d'hydrogène et Z représente un groupe —$CH_2COOH$.

2. Composés suivant la revendication 1, caractérisé en ce qu'ils sont représentés par la formule générale (IV) ci-après:

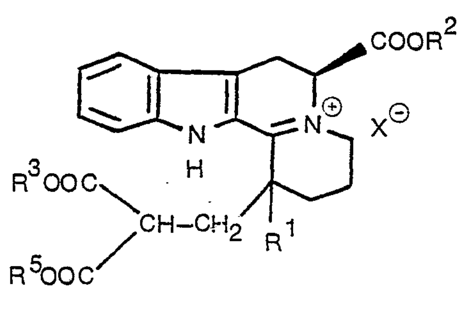

(IV)

sont tels que définis dans la revendication 1.

3. Composés suivant la revendication 1, caractérisés en ce qu'ils sont représentés par la formule générale (V), ci-après:

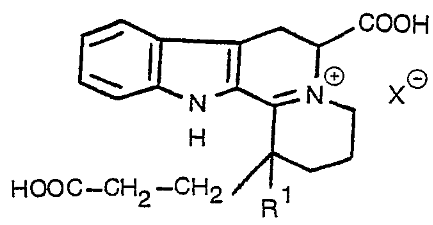

(V)

où $R^1$ et X sont tels que définis dans la revendication 1.

4. Composés suivant la revendication 1, caractérisés en ce qu'ils sont représentés par la formule générale (VI) ci-après:

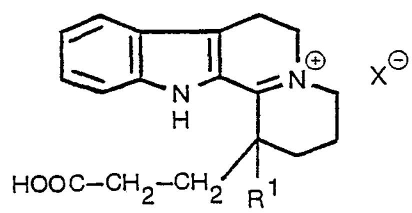

(VI)

où $R^1$ et X sont tels que définis dans la revendication 1.

5. Composé suivant la revendication 1, caractérisé en ce qu'il est choisi parmi les composés suivants:

le perchlorate de (—)-1α-éthyl-1β-(2',2'-diéthoxycarbonyléthyl)-6β-méthoxycarbonyl-1,2,3,4,6,7-hexahydro-12H-indolo(2,3-a)quinolizin-5-ium.

(+)-1α-éthyl-1β-carboxyéthyl-6-carboxy-1,2,3,4,6,7-hexahydro-12H-indolo(2,3-a)quinolizin-5-ium, et

le perchlorate de (—)-1α-éthyl-1β-carboxyéthyl-1,2,3,4,6,7-hexahydro-12H-indolo(2,3-a)quinolizin-5-ium.

6. Procédé pour la préparation de composés de formule générale (IV) telle que définie dans la revendication 2, caractérisé en ce que l'on fait réagir un sel optiquement actif de 6-alcoxy-carbonyl-hexahydroindoloquinolizinium de formule (II) ci-après:

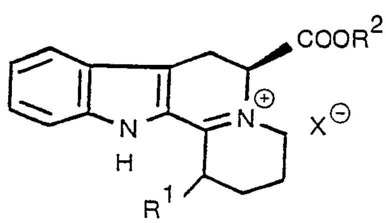

(II)

(où $R^1$, X et $R^2$ sont tels que définis dans la revendication 1) avec un diester d'acide méthylène-malonique de formule (III) ci-après:

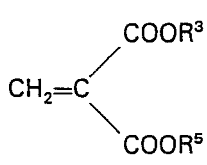

(III)

(où $R^3$ et $R^5$ sont tels que définis dans la revendication 1), en présence d'un catalyseur fortement basique.

7. Procédé pour la préparation des composés de formule générale (V) telle que définie dans la revendication 3, caractérisé en ce que l'on soumet un composé de formule (IV), telle que défini dans la revendication 2, à une hydrolyse acide et ensuite alcaline.

8. Procédé pour la préparation des composés de formule générale (VI) telle que définie dans la revendication 4, caractérisé en ce que l'on soumet un composé de formule (V) telle que définie dans la revendication 3, à une décarboxylation sélective par laquelle on obtient le composé désiré représenté par la formule (VI).

9. Utilisation d'un composé de formule (VI), telle que définie dans la revendication 4, dans un procédé pour la préparation de composés de formule générale (VII) ci-après:

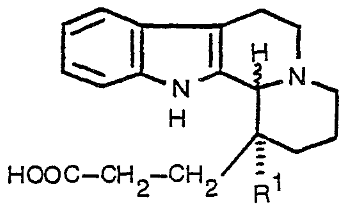

(VII)

(où $R^1$ est tel que défini dans la revendication 1), caractérisée en ce que l'on sature le composé de formule (VI) en obtenant ainsi le composé désiré représenté par la formule (VII).

10. Procédé suivant la revendication 9, caractérisé en ce que l'on cyclise ensuite le composé de formule (VII) pour fournir un composé de formule (Ia):

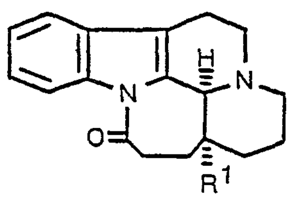

(Ia)

(où $R^1$ est tel que défini dans la revendication 1).

11. Utilisation d'un composé de formule (VI) telle que définie dans la revendication 4, dans un procédé pour la préparation des composés de formule générale (VIa) ci-après:

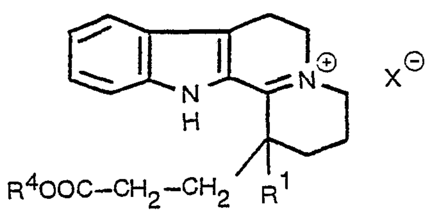

(VIa)

(où $R^1$ et X sont tels que définis dans la revendication 1 et $R^4$ représente un groupe alcoyle en $C_{1-4}$), caractérisée en ce que l'on estérifie le composé de formule (VI).

12. Procédé suivant la revendication 11, caractérisé en ce que l'on sature ensuite le composé de formule (VIa) pour obtenir un composé de formule (VIII) ci-après

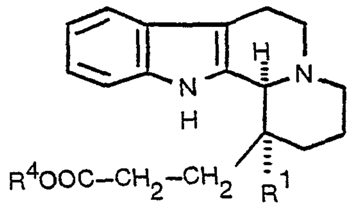

(VIII)

(où $R^1$ est tel que défini dans la revendication 1 et $R^4$ est tel que défini dans la revendication 11).

13. Procédé suivant la revendication 9, caractérisé en ce que l'on estérifie le composé de formule (VII) pour obtenir un composé de formule (VIII), telle que définie dans la revendication 12.

14. Procédé suivant la revendication 12 ou la revendication 13, caractérisé en ce que l'on soumet ensuite le composé de formule (VIII) à une cyclisation, fournissant ainsi un composé de formule (Ia) telle que définie dans la revendications 10.